# EUROPEAN PATENT APPLICATION

(11) **EP 4 292 603 A1**
(43) Date of publication of application: **20.12.2023**
(21) Application number: 22855425.9
(22) Date of filing: 09.08.2022
(51) Int. Cl.: A61K 38/17, A61K 39/395, C07K 19/00, A61P 37/02

(54) **METHOD FOR TREATING IGA NEPHROPATHY WITH TACI-FC FUSION PROTEIN**

(30) Priority: 10.08.2021 CN 202110915873
(71) Applicant: RemeGen Co., Ltd., Shandong 264006 (CN)
(72) Inventor: FANG, Jianmin, Beijing Middle Road, Yantai Development Zone, Yantai District, China (Shandong) Pilot Free Trade Zone Yantai, Shandong 264006 (CN); WANG, Wenxiang, Beijing Middle Road, Yantai Development Zone, Yantai District, China (Shandong) Pilot Free Trade Zone Yantai, Shandong 264006 (CN)
(74) Representative: HGF
(86) International application number: PCT/CN2022/111112
(87) International publication number: WO 2023/016444

(57) **Abstract**

The present invention relates to a drug for treating IgA nephropathy with a TACI-Fc fusion protein, a dose regimen, an administration interval and an application method. Results show that the urine protein levels of patients in a treatment group are significantly reduced compared with a baseline, and are statistically significant compared with a placebo group. In addition, a significant difference between the treatment group and the placebo control group in multiple secondary endpoints of clinical trials also exists.

## Description

### FIELD

The present disclosure relates to a drug for treating IgA nephropathy, a dosage regimen, an administration interval and an administration route.

### BACKGROUND

IgA nephropathy (i.e. immunoglobulin A nephropathy, IgAN), as a glomerulonephritis caused by immune complexes, is manifested as hematuria, proteinuria and progressive renal failure in different degrees. The pathogenesis of the disease is very complicated. It is believed that genetic, environmental and immune factors jointly lead to the occurrence of IgA nephropathy. It is mainly believed that multiple factors lead to the excessive production of abnormally glycosylated poly IgA1 molecules, which deposit in the renal thylakoid region, inducing an inflammatory response and excessive complement activation in the thylakoid region, thereby resulting in the occurrence and progression of the disease. Patients eventually develop renal failure or end-stage renal disease, with up to 50% requiring dialysis or a kidney transplant. According to information from Frost & Sullivan, the number of patients with IgA nephropathy worldwide increased from 8.8 million in 2015 to 9.3 million in 2020 (including 2.2 million in China). It is estimated that the total number of patients with IgA nephropathy worldwide will reach 9.7 million (including 2.3 million in China) in 2025 and 10.2 million (including 2.4 million in China) in 2030. Therefore, there is a huge clinical need in the field of IgA nephropathy treatment worldwide.

However, currently, there is no specific therapy or biological drug approved for IgA nephropathy in the world. At present, the standard therapy is blockade of renal-angiotensin-aldosterone system and immunosuppression, including using corticosteroid, which usually has severe toxic side effects. As of July 20, 2021, there are only 11 new biological drugs in the clinical trials for IgA nephropathy in the world (see Table 1).

**Table 1 New biological drugs undergoing clinical research for IgA nephropathy in the world**

| **Drug name** | **Company** | **Current development status in the world** |
|---|---|---|
| Telitacicept | RemeGen Biopharmaceutical (Yantai) Co., Ltd. | Phase II clinical trial |
| Ravulizumab | Alexion Pharmaceuticals Inc | Phase II clinical trial |
| Narsoplimab | Omeros | Phase III clinical trial |
| AVB-S6-500 | Aravive Biologies | Phase II clinical trial |
| Atacicept | Zymogenetics | Phase II clinical trial |
| VIS-649 | Visterra | Phase II clinical trial |
| BION-1301 | Aduro Biotech | Phase II clinical trial |
| Blisibimod | Anthera | Phase III clinical trial |
| Valziflocept | Suppremol | Phase II clinical trial |
| CCL2-LPM | Osprey Pharmaceuticals | Phase I clinical trial |
| CR-002 (CuraGen) | Curagen; Amgen | Phase I clinical trial |

Therefore, there is a huge clinical need in the field of IgA nephropathy treatment, both in China and in the world.

### SUMMARY

According to a deep analysis of clinical data, the TACI-Fc fusion protein provided by the present invention exhibits significant technical effects in the treatment of a patient with IgA nephropathy and cancer.

Specifically, the present invention provides a method for treating IgA nephropathy, comprising administering a therapeutically effective amount of a TACI-Fc fusion protein to a subject in need thereof, wherein the TACI-Fc fusion protein comprises:
(i) a TACI extracellular region or a fragment thereof binding to Blys and/or APRIL; and
(ii) a human immunoglobulin constant region.

Further, the TACI extracellular region or the fragment thereof binding to Blys and/or APRIL comprises an amino acid sequence set forth in SEQ ID NO: 1.
**SEQ ID NO: 1**

Further, the human immunoglobulin constant region comprises an amino acid sequence set forth in SEQ ID NO: 2 or an amino acid sequence having at least 90%, at least 91%, at least 92%, at least 93%, at least 95%, at least 96%, at least 97%, at least 98%, or at least 99% identity to SEQ ID NO: 2.
**SEQ ID NO: 2**

Further, the human immunoglobulin constant region comprises one or more amino acid modifications at positions 3, 8, 14, 15, 17, 110, 111 or 173 of SEQ ID NO: 2, wherein the modification is preferably substitution, deletion or insertion of amino acid.

Further, the substitution is selected from the group consisting of P3T, L8P, L14A, L15E, G17A, A110S, P111S and A173T.

Further, the human immunoglobulin constant region comprises an amino acid sequence set forth in SEQ ID NO: 3.
**SEQ ID NO: 3**

Further, the human immunoglobulin is IgG1.

Further, the amino acid sequence of TACI-Fc fusion protein is set forth in SEQ ID NO: 4.
**SEQ ID NO: 4**

Further, the TACI-Fc fusion protein is Telitacicept.

Further, the TACI-Fc fusion protein is administered at a dose ranging from 160 to 240 mg each time; in some specific embodiments, the TACI-Fc fusion protein is administered at a dose of 160 mg each time; in other specific embodiments, the TACI-Fc fusion protein is administered at a dose of 240 mg each time.

Further, the TACI-Fc fusion protein is administered 2-4 times per month.

Further, the TACI-Fc fusion protein is administered continuously for about 2-50 weeks.

Further, the TACI-Fc fusion protein is administered by subcutaneous injection, intramuscular injection, oral or intravenous administration.

Further, the IgA nephropathy is primary IgA nephropathy.

Further, the subject in need thereof is an adult or a child.

The present invention further provides a method for treating IgA nephropathy, comprising administering a pharmaceutical composition comprising a TACI-Fc fusion protein to a subject in need thereof, wherein the pharmaceutical composition further comprises a pharmaceutically acceptable carrier.

The present invention further provides use of a TACI-Fc fusion protein in the manufacture of a medicament for treating IgA nephropathy.

In a clinical study carried out by the present invention, the results show that subjects in the Telitacicept treatment group have significantly lower urine protein levels compared with the baseline, and the difference was statistically significant compared with the placebo group. In addition, there is also a significant difference between the treatment group and the placebo control group in multiple secondary endpoints of clinical trials.

### BRIEF DESCRIPTION OF DRAWINGS

FIG. 1 shows the diachronic changes of eGFR;
FIG. 2 shows the diachronic changes of UACR;
FIG. 3 shows the diachronic changes of red blood cell count in the urine;
FIG. 4 shows the diachronic changes of IgA level;
FIG. 5 shows the diachronic changes of IgG level;
FIG. 6 shows the diachronic changes of IgM level;
FIG. 7 shows the diachronic changes of B lymphocyte (CD19+) count.

### DETAILED DESCRIPTION

Unless otherwise defined, all scientific and technical terms used herein have the same meaning as understood by those of ordinary skill in the art. For definitions and terms in the art, those skilled can make a reference to Current Protocols in Molecular Biology (Ausubel).

The present invention provides use of a TACI-Fc fusion protein (also named "TACI-immunoglobulin fusion protein", which can be used interchangeably in the present invention) in the treatment of IgA nephropathy. The subject involved in the present invention is preferably a mammal, such as a human; more preferably, the subject involved in the present invention is an adult or a child.

The TACI-immunoglobulin fusion protein provided by the present invention comprises: 1) a polypeptide containing a domain that is at least partially identical to the extracellular domain of TACI or a fragment thereof capable of binding BlyS and/or APRIL; and 2) a human immunoglobulin constant region. U.S. Patent Nos. 5,969,102, 6,316,222, and 6,500,428 and U.S. Patent Applications 09/569,245 and 09/627,206 (the contents thereof are incorporated herein by reference), disclose the extracellular domain of TACI and specific fragments of TACI extracellular domain capable of interacting with TACI ligands, wherein the TACI ligands include BlyS and APRIL. Chinese Patent Publication No. CN101323643A discloses a TACI-Fc fusion protein comprising a fragment of the 13th-118th amino acids of the TACI extracellular domain.

The term "TACI" used in the present invention refers to a transmembrane activator and CAML interactor, which is a member of the tumor necrosis factor receptor superfamily. The term "BLys" used in the present invention refers to a B lymphocyte stimulator, which is a member of the TNF ligand superfamily existing in two forms of membrane-bound form and soluble form, expressed on the surface of bone marrow cells, and can selectively stimulate the proliferation of B lymphocytes and the production of immunoglobulin. The term "APRIL" (a proliferation-inducing ligand) used in the present invention refers to a tumor necrosis factor (TNF) analogue, which can stimulate the proliferation of primitive B cells and T cells in the body, promote the accumulation of B cells and increase its content in spleen. APRIL can specifically bind to TACI and BCMA, and the binding can prevent APRIL from binding to B cells and inhibit the proliferative response of primitive B cells stimulated by APRIL. Moreover, APRIL can compete with BLys for binding to receptors BCMA and TACI.

The term "immunoglobulin" used in the present invention refers to an animal protein with antibody activity, preferably a human immunoglobulin. In some embodiments, the "immunoglobulin" may be IgG, IgM, IgE, IgD or IgA, preferably, the immunoglobulin is IgG, more preferably, the immunoglobulin is IgG1 or IgG4.

The term "human immunoglobulin constant region" used in the present invention specifically refers to the heavy chain constant region of a human immunoglobulin. In one embodiment, the "human immunoglobulin constant region" is a complete human immunoglobulin heavy chain constant region; in another embodiment, the "human immunoglobulin constant region" is a fragment having partial amino acid sequence of a "complete human immunoglobulin heavy chain constant region", *i.e.* "human immunoglobulin heavy chain constant region fragment". Taking human immunoglobulin IgG1 as an example, the "complete human immunoglobulin heavy chain constant region" comprises a CH1 domain, a hinge region, a CH2 domain and a CH3 domain. The "human immunoglobulin heavy chain constant region fragment" may have various structure combinations, including but not limited to the following: a complete or partial amino acid sequence of CH1 domain, a complete or partial amino acid sequence of hinge region, a complete or partial amino acid sequence of CH2 domain, a complete or partial amino acid sequence of CH3 domain, a complete or partial amino acid sequence of CH1 domain and a complete or partial amino acid sequence of hinge region, a complete or partial amino acid sequence of CH1 domain and a complete or partial amino acid sequence of CH2 domain, a complete or partial amino acid sequence of CH1 domain and a complete or partial amino acid sequence of CH3 domain, a complete or partial amino acid sequence of hinge region and a complete or partial amino acid sequence of CH2 domain, a complete or partial amino acid sequence of hinge region and a complete or partial amino acid sequence of CH3 domain, a complete or partial amino acid sequence of CH2 domain and a complete or partial amino acid sequence of CH3 domain, a complete or partial amino acid sequence of CH1 domain and a complete or partial amino acid sequence of hinge region as well as a complete or partial amino acid sequence of CH2 domain, a complete or partial amino acid sequence of CH1 domain and a complete or partial amino acid sequence of hinge region as well as a complete or partial amino acid sequence of CH3 domain, a complete or partial amino acid sequence of hinge region and a complete or partial amino acid sequence of CH2 domain as well as a complete or partial amino acid sequence of CH3 domain. In some preferred embodiments of the present invention, the "human immunoglobulin constant region" refers specifically to a fragment of the human immunoglobulin heavy chain constant region, which preferably comprises a partial amino acid sequence of hinge region and a complete amino acid sequence of CH2 domain as well as a complete amino acid sequence of CH3 domain. In some preferred embodiments, the "human immunoglobulin constant region" involved in the present invention exemplarily comprises an amino acid sequence set forth in SEQ ID NO: 2 or SEQ ID NO: 3 or a sequence having identity therewith.

The immunoglobulin moiety of the TACI-immunoglobulin fusion protein provided by the present invention is preferably IgG1. The preferred IgG1 heavy chain constant region of the present invention comprises an IgG1 Fc fragment containing CH2 and CH3 domains, which may be a wild-type IgG1 Fc fragment or a mutated IgG1 Fc fragment.

As an alternative embodiment, the immunoglobulin constant region provided by the present invention may be introduced with one or more amino acid modifications, such as substitution (i.e. mutation), addition (i.e. insertion) or deletion.

The term "therapeutically effective amount" used in the present invention refers to the commonly used amount of a drug, which is the effective amount range commonly used clinically, generally an amount between the minimum effective amount and the maximum amount. The minimum effective amount refers to the minimum amount of the drug that can cause pharmacological effects. The maximum amount refers to the maximum therapeutic amount, i.e., the limit of safe medication, beyond which poisoning may occur.

The term "Telitacicept" used in the present invention (or "Tai'ai", which can be used interchangeably in the present invention) refers to a TACI-Fc fusion protein, having an INN name of Telitacicept, and an amino acid sequence set forth in SEQ ID NO: 4, or referring to https://extranet.who.int/soinn/mod/page/view.php?id=137&inn_n=10932.

The three-letter codes and one-letter codes of amino acids used in the present invention are as described in J.biol.chem, 243, p3558 (1968). There are many ways of numbering amino acid positions, such as Kabat numbering system, EU numbering system, and sequence numbering. In the present invention, the amino acid positions are numbered using "sequence numbering", for example, the "position 3, 8, 14, 15, 17, 110, 111 or 173 of SEQ ID NO: 2" described in the present invention refers to the 3rd amino acid, the 8th amino acid and so on of SEQ ID NO: 2; for example, the "P3T" described in the present invention refers to the mutation of the 3rd amino acid of SEQ ID NO:2, from the previous "P" to "T", and "L8P" refers to the mutation of the 8th amino acid of SEQ ID NO:2, from the previous "L" to "P", and so on.

The term "pharmaceutical composition" used in the present invention specifically refers to a preparation comprising a TACI-Fc fusion protein, which may be in different dosage forms, such as an injectable solution or a suspension; or it may be in a pre-injection solid form suitable for dissolution in a liquid carrier as a solution or suspension (e.g. a lyophilized composition and sterile water with preservatives for reconstitution); or it may be prepared in a form for topical administration, such as ointment, cream or powder; or it may be prepared for oral administration; or it may be a tablet or capsule, or a spray, or a syrup (optionally flavored). Typically, it can be prepared as an injectable liquid solution or suspension, or as a pre-injection solid form suitable for dissolution in a liquid carrier as a solution or suspension.

The TACI-Fc fusion protein of the present invention may be administered through various routes, including but not limited to oral administration, intravenous injection, intramuscular injection, intraarterial injection, intramedullary injection, intraperitoneal injection, intrathecal injection, intracardiac administration, transdermal administration, transdermal administration, topical administration, subcutaneous administration, intranasal administration, enteral administration, sublingual administration, vaginal administration, rectal administration, etc.

The TACI-Fc fusion protein or pharmaceutical composition thereof provided by the present invention is prepared and stored in lyophilized form, aseptic form and solution. The term "treatment" used in the present invention is related to a given disease or condition, including but not limited to: inhibiting the disease or symptom, such as preventing the development of the disease or symptoms; alleviating the disease or symptoms, such as causing the regress of the disease or symptoms; or alleviating the symptoms caused by the disease or symptoms, such as alleviating, preventing or treating the symptoms caused by the disease or symptoms.

Embodiments of the present invention will be described in detail below in conjunction with examples. However, those skilled in the art will understand that the following examples are only intended to illustrate the present invention, and should not be construed as limiting the scope of the present invention.

### Example 1 Phase II clinical trial of Telitacicept in treating IgA nephropathy

1. Inclusion criteria
1) Pathological biopsy-confirmed IgA nephropathy;
2) Age≥18 years old, ≤70 years old, male or female;
3) During the screening period, at least one of the 24-hour urine protein in Visit 1 and Visit 2 was≥0.75 g/24 h, and the 24-hour urine protein in Visit 3 was≥0.75 g/24h;
4) Measured glomerular filtration rate or estimated GFR (using CKD-EPI formula)>35 mL/min per 1.73 m²;
5) Subjects had received basic treatment regimen including ACEI/ARB drugs for 12 weeks before randomization, and the amount of ACEI/ARB drugs (within the maximum tolerance range) was stable within 4 weeks before randomization.

2. Test grouping
(1) Test group

| **NO.** | **Name** | **Administration method** |
|---|---|---|
| 1 | Recombinant human B lymphocyte stimulator receptor-antibody fusion protein for injection (Telitacicept) | Administration route and amount: A lyophilized preparation for injection; 80 mg/ piece; subcutaneous injection: once a week, 160 mg each time, 24-week course of treatment. |
| 2 | Recombinant human B lymphocyte stimulator receptor-antibody fusion protein for injection (Telitacicept) | Administration route and amount: A lyophilized preparation for injection; 80 mg/ piece; subcutaneous injection: once a week, 240 mg each time, 24-week course of treatment. |

(2) Control group

| **NO.** | **Name** | **Administration method** |
|---|---|---|
| **1** | Placebo | Administration route and amount: A lyophilized preparation for injection with no active ingredients (including histidine hydrochloride, arginine hydrochloride, mannitol and sucrose); specification: 80 mg/piece; subcutaneous injection: once a week, 24-week course of treatment. |

3. Endpoint indicator
(1) Primary endpoint indicator (i.e. analysis of primary efficacy indicator)

| **NO.** | **Indicator** | **Evaluation time** | **Selection of endpoint indicator** |
|---|---|---|---|
| 1 | Change in 24-hour urine protein at the 24th week compared with baseline. | The 24th week | Indicator of effectiveness |

(2) Secondary endpoint indicator (i.e. analysis of secondary indicator)

| **NO.** | **Indicator** | **Evaluation time** | **Selection of endpoint indicator** |
|---|---|---|---|
| 1 | Estimated change in glomerular filtration rate (eGFR) compared with baseline | The 4th, 8th, 12th, 16th, and 24th weeks | Indicator of effectiveness |
| 2 | Change in urine protein/creatinine ratio (UPCR) and/or urinary albumin/creatinine ratio (UACR) compared with baseline. | The 4th, 8th, 12th, 16th, and 24th weeks | Indicator of effectiveness |
| 3 | Change in urinary red blood cell count compared with baseline. | The 4th, 8th, 12th, 16th, and 24th weeks | Indicator of effectiveness |
| 4 | Change in immunological indicators (IgA, IgG, IgM, C3, C4, B lymphocytes) compared with baseline. | The 4th, 8th, 12th, 16th, and 24th weeks | Indicator of effectiveness |
| 5 | Incidence and severity of adverse events. | The 4th, 8th, 12th, 16th, and 24th weeks | Indicator of safety |

4. Statistical methods and analysis data sets

Continuous variables were described by means, standard deviations, medians, minimum and maximum values, and count and rank data were described by frequencies and percentages. All statistical tests were two-sided tests, and p less than or equal to 0.05 (p≤0.05) was considered statistically significant. Two data sets, full analysis set (FAS) and per protocol set (PPS) were used for efficacy analysis, and safety data set (SS) is used for safety analysis.

A total of 44 subjects were included in the full analysis set (FAS) of this study, including 14 subjects in the placebo group, 16 subjects in the Telitacicept 160 mg group, and 14 subjects in the Telitacicept 240 mg group. The data of safety data set (SS) was the same as that of the FAS set. In the per protocol set (PPS), 2 subjects in the placebo group who quit voluntarily were excluded, and 42 subjects were finally included, including 12 subjects in the placebo group, 16 subjects in the Telitacicept 160 mg group, and 14 subjects in the Telitacicept 240 mg group.

### 5. Main results

### (1) Analysis of primary efficacy indicators

At the 24th week of administration, the average 24-hour urine protein level of the subjects in the placebo group was basically unchanged, showing a slight decrease of 0.03 g/24 h (2.62%); the average urine protein level of the subjects in Telitacicept 240 mg group decreased by 0.89 g/24 h (49.29%) after the administration, and the average urine protein level of the subjects in Telitacicept 160 mg group decreased by 0.32 g/24 h (24.71%) after the administration. In the Telitacicept 240 mg group, the 24-hour urine protein level of 13 subjects (92.86%) decreased compared with the baseline level, wherein the greatest three decreases of the subjects were1.89 g/24 h, 1.77 g/24 h and 1.54 g/24 h, respectively. At the 24th week of administration, the 24-hour urine protein level of 11 subjects (68.75%) in the Telitacicept 160 mg group decreased compared with the baseline level, wherein the greatest decrease was 1.40 g/24 h compared with the baseline level.

### (2) Analysis of secondary efficacy indicators

### A. Diachronic analysis of estimated glomerular filtration rate (eGFR)

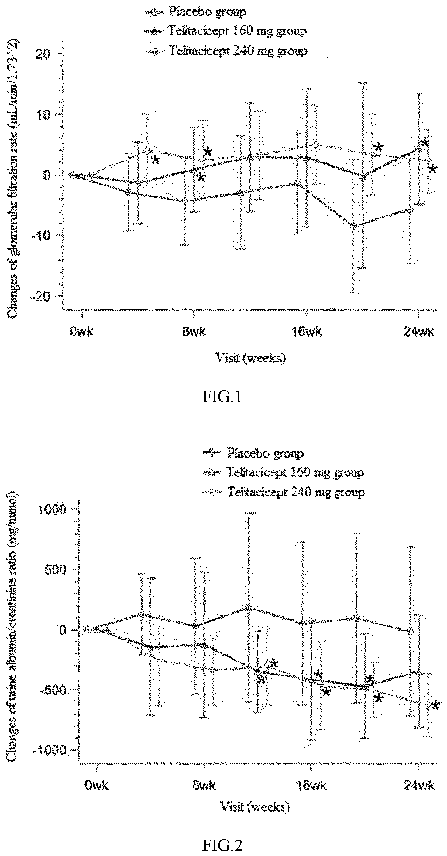

At the 24th week of administration, the average levels of estimated glomerular filtration rate (eGFR) of subjects in the Telitacicept 160 mg group and Telitacicept 240 mg group increased by 4.32 mL/min/1.73 m² and 2.33 mL/min/1.73 m² respectively, while the average level in the placebo group decreased by 5.70 mL/min/1.73 m². Analysis of variance (ANOVA) shows that the changes of eGFR in the Telitacicept 160 mg group and Telitacicept 240 mg group were significantly different from that of the placebo group, with p being 0.002 and 0.015, respectively. The diachronic eGFR changes of the subjects in each group are shown in FIG. 1.

### B. Diachronic analysis of urinary albumin-creatinine ratio (UACR)

In this test, UACR data was collected selectively, and the UACR data of 37 subjects were collected during the test (13 subjects in the placebo group; 14 subjects in the Telitacicept 160 mg group; 10 subjects in the Telitacicept 240 mg group). As shown in FIG. 2, during the administration, the average UACR levels of the two Telitacicept treatment groups showed a continuous decline. The results of ANOVA analysis confirmed that there was a statistically significant difference between the decline of UACR of subjects in the two Telitacicept administration groups and the change of UACR of subjects in the placebo group (p≤0.05). At the 12th week, 16th week and 20th week of administration, there was a statistical difference in the 24-hour urine protein changes between the subjects in the two Telitacicept administration groups and the subjects in the placebo group. Compared with the baseline level, at the 24th week of administration, the average UACR of subjects in the Telitacicept 160 mg group and Telitacicept 240 mg group decreased by 347.81 mg/g and 627.55 mg/g, respectively, and the average UACR of subjects in the placebo group decreased by 16.77 mg/g.

### C. Diachronic analysis of red blood cell counts in the urine

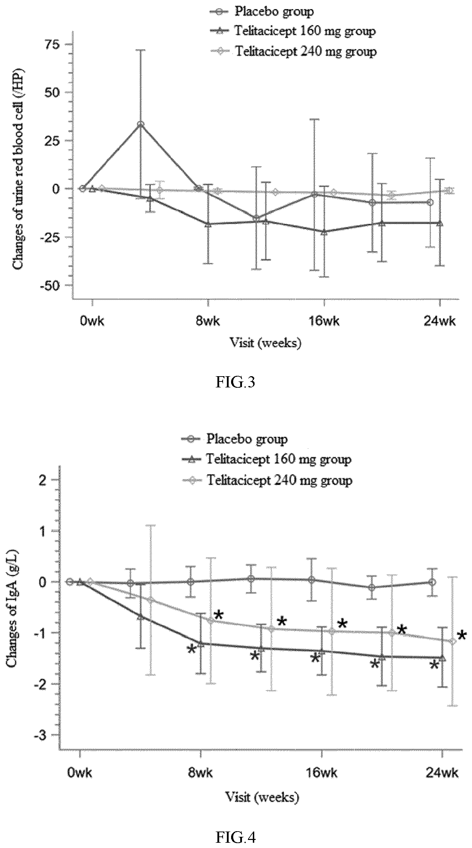

The number of the red blood cells in the urine in each group fluctuated little before and after the administration. The results of ANOVA analysis show that there was no statistical difference in the changes of red blood cells in the urine among the subjects in all groups. The diachronic changes of urinary red blood cell counts of the subjects in each group are shown in FIG. 3.

### D. Diachronic analysis of immunoglobulin A (IgA)

Since the start of the administration, the level of immunoglobulin A (IgA) of subjects in the Telitacicept administration groups decreased continuously until the 24th week of administration. The immunoglobulin A (IgA) of the two Telitacicept treatment groups started to be statistically significantly different from that of the placebo group since the 8th week of administration (p<0.001), and the statistical difference continued until the 24th week of administration. Compared with the baseline, at the 24th week of administration, the average IgA level of subjects in the placebo group changed slightly, showing a decrease of 0.01 g/L (with a decrease rate of 1.11%), while the average IgA level of subjects in the Telitacicept 160 mg group decreased by 1.48 g/L (45.75%), and the average IgA level of subjects in the Telitacicept 240 mg group decreased by 1.17 g/L (15.83%). The diachronic changes of the average IgA level of the subjects in each group are shown in FIG. 4.

### E. Diachronic analysis of immunoglobulin G (IgG)

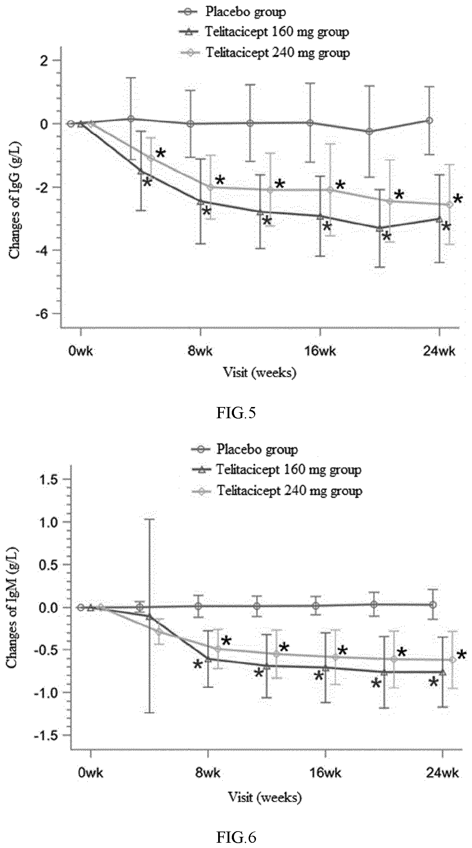

After administration, the immunoglobulin G (IgG) level of subjects in the Telitacicept administration group decreased continuously. ANOVA analysis shows that the changes of immunoglobulin G (IgG) in the two Telitacicept administration groups started to be statistically significantly different from that of the placebo group since the 4th week (p<0.001), and the statistical difference continued until the 24th week of administration. At the 24th week of administration, compared with the baseline, the IgG level of subjects in the placebo group changed little, with an average increase of 0.10 g/L (0.22%). The IgG level of subjects in the Telitacicept 160 mg group decreased by 3.00 g/L (26.16%) and the IgG level of subjects in the Telitacicept 240 mg group decreased by 2.56 g/L (24.55%). The diachronic changes of the average IgG level of subjects in each group are shown in FIG. 5.

### F. Diachronic analysis of immunoglobulin M (IgM)

Since the start of administration, the level of immunoglobulin M (IgM) of subjects in the Telitacicept administration group decreased continuously. The statistical difference between the changes of the Telitacicept administration groups and the changes of the placebo group lasted from the 8th week of administration to the 24th week of administration. Compared with the baseline level, at the 24th week of administration, the average IgM level of subjects in the placebo group remained basically unchanged, showing an increase of 0.03 g/L (0.63%). The average IgM level of subjects in the Telitacicept 160 mg group decreased by 0.76 g/L (64.28%), and the average IgM level in the Telitacicept 240 mg group decreased by 0.62 g/L (65.34%). Statistical analysis shows that the changes in IgM in the two Telitacicept administration groups started to be statistically significantly different from that in the placebo group since the 8th week (p<0.001), and the statistical difference continued until the 24th week of administration. The diachronic changes of the average IgM levels of subjects in each group are shown in FIG. 6.

### G. Diachronic analysis of B lymphocyte (CD19⁺) count

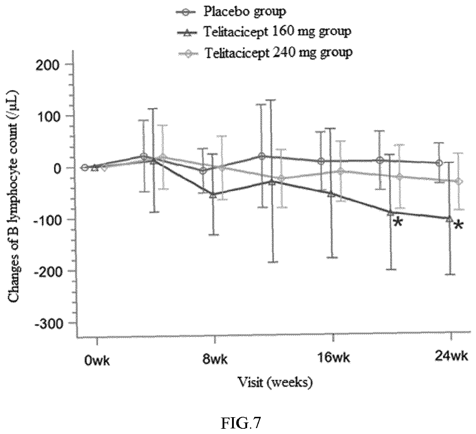

At the 24th week of administration, compared with the baseline, the average B lymphocyte (CD19⁺) level of subjects in the placebo group decreased by 0.32 cells/µl (1.18%); the average B lymphocyte (CD19⁺) level of subjects in the Telitacicept 160 mg group decreased by 108.36 cells/µl (26.23%); the average B lymphocyte (CD19⁺) level of subjects in the Telitacicept 240 mg group decreased by 36.72 cells/µl (21.40%). During the whole trial, the B lymphocyte (CD19⁺) count of subjects in the Telitacicept 240 mg group was not significantly different from that in the placebo group (p>0.05). At the 20th and 24th weeks of administration, the B lymphocyte (CD19⁺) count of subjects in the Telitacicept 160 mg group was significantly different from that in the placebo group (p≤0.05). The specific changes are shown in FIG. 7.

### H. Safety evaluation

This study preliminarily revealed the good safety of Telitacicept in the treatment of IgA nephropathy. During the trial, there were no deaths and no cases of withdrawal due to adverse reactions (AE). Except for 2 subjects who withdrew early due to their own reasons (unable to receive the drug as planned), the rest of the subjects were all administered as planned. There were no significant differences in the incidence of treatment emergent adverse events (TEAE), serious adverse events (SAE) and serious adverse reactions (SADR) among the groups (P>0.05). The difference in the incidence of ADR among the groups was statistically significant (p<0.05). The adverse reactions (ADR) of subjects in the administration groups were mainly injection site reactions, which had an intensity of below grade 3.

To sum up, the test data show that after 24 weeks of administration, the 24-hour urine protein levels of subjects in the Telitacicept 240 mg group and Telitacicept 160 mg group were lower than the baseline. Among them, the average 24-hour urine protein of subjects in the Telitacicept 240 mg group decreased by 0.89 g/24 h (49.29%); the average 24-hour urine protein of subjects in the Telitacicept 160 mg group decreased by 0.32 g/24 h (24.71%). From the 16th week to the 24th week of administration, the urine protein level of subjects in the Telitacicept 240 mg group decreased compared with the placebo group, and the difference was statistically significant. From the 16th week to the 20th week of administration, the 24-hour urine protein of subjects in the Telitacicept 160 mg group decreased compared with the placebo group, and the difference was statistically significant. Comparing the data before and after administration, the average estimated glomerular filtration rate (eGFR), a long-term renal indicator, of subjects in the two Telitacicept administration groups increased, and the average level of immunoglobulins (IgA, IgG, and IgM) decreased significantly. Compared with the placebo group, the differences in the changes of the above indicator among the groups were also statistically significant. Since there is currently no recognized targeting drug for the treatment of IgA nephropathy, there is currently no clinical data of positive drugs for horizontal comparison. However, the results obtained in the study are superior to most of the clinical trial data of drugs for IgA treatment in the same period. Therefore, the study reveals the effectiveness of Telitacicept 240 mg/week and Telitacicept 160 mg/week in the treatment of IgA nephropathy.

In addition, the trial data also reveals that Telitacicept has good safety in the treatment of IgA nephropathy. During the trial, there were no serious adverse events, no subjects who withdrew from the trial due to adverse events, and no deaths.

The above results show that the urine protein levels of subjects in the Telitacicept treatment groups were significantly lower than the baseline, and were statistically significantly different from that of the placebo group. In addition, there were also significant differences in multiple secondary endpoints of the clinical trial between the treatment groups and the placebo control group. Based on the above results and analysis, this study reveals the effectiveness and good safety of Telitacicept (240 mg/week) and Telitacicept (160 mg/week) in the treatment of IgA nephropathy.

The above descriptions are only preferred embodiments, which are only examples and do not limit the combination of features necessary to implement the present invention. The headings provided are not intended to limit the various embodiments of the present invention. Terms such as "comprise," "contain," and "include" are not intended to be limiting. In addition, unless otherwise stated, the absence of a numerical modifier includes the plural, and "or" means "and/or". Unless otherwise defined herein, all technical and scientific terms used herein have the same meaning as commonly understood by those of ordinary skill in the art.

All publications and patents mentioned in the present application are incorporated herein by reference. Various modifications and variations of the methods and compositions described in the present invention without departing from the scope and spirit of the present invention will be apparent to those skilled in the art. Although the present invention has been described with specific preferred embodiments, it should be understood that the present invention as claimed should not be unduly limited to these specific embodiments. In fact, various modifications of the described modes for implementing the present invention which are apparent to those skilled in the relevant fields are intended to be included within the scope of the following claims.

## Claims

1. A method for treating IgA nephropathy, comprising administering a therapeutically effective amount of a TACI-Fc fusion protein to a subject in need thereof, wherein the TACI-Fc fusion protein comprises:
(i) a TACI extracellular region or a fragment thereof binding to Blys and/or APRIL; and
(ii) a human immunoglobulin constant region.

2. The method according to claim 1, wherein the TACI extracellular region or the fragment thereof binding to Blys and/or APRIL comprises an amino acid sequence set forth in SEQ ID NO: 1.

3. The method according to claim 2, wherein the human immunoglobulin constant region comprises an amino acid sequence set forth in SEQ ID NO: 2 or an amino acid sequence having at least 90%, at least 91%, at least 92%, at least 93%, at least 95%, or at least 96% identity to SEQ ID NO: 2.

4. The method according to claim 3, wherein the human immunoglobulin constant region comprises one or more modifications of amino acid at positions 3, 8, 14, 15, 17, 110, 111 or 173 of SEQ ID NO: 2.

5. The method according to claim 4, wherein the modification is substitution, deletion or insertion of amino acid.

6. The method according to claim 5, wherein the substitution is selected from the group consisting of P3T, L8P, L14A, L15E, G17A, A110S, P111S and A173T.

7. The method according to claim 6, wherein the human immunoglobulin constant region comprises an amino acid sequence set forth in SEQ ID NO: 3.

8. The method according to claim 7, wherein the human immunoglobulin is IgG1.

9. The method according to claim 8, wherein the TACI-Fc fusion protein comprises an amino acid sequence set forth in SEQ ID NO: 4.

10. The method according to claim 9, wherein the TACI-Fc fusion protein is Telitacicept.

11. The method according to any one of claims 1-10, wherein the TACI-Fc fusion protein is administered at a dose ranging from 160 to 240 mg each time, more preferably 160 mg or 240 mg each time.

12. The method according to any one of claims 1-10, wherein the TACI-Fc fusion protein is administered 2-4 times per month and/or administered continuously for about 2-50 weeks.

13. The method according to any one of claims 1-10, wherein the TACI-Fc fusion protein is administered by subcutaneous injection, intramuscular injection, oral or intravenous administration.

14. The method according to claim 1, wherein the IgA nephropathy is primary IgA nephropathy.

15. A method for treating IgA nephropathy, comprising administering a pharmaceutical composition comprising a TACI-Fc fusion protein to a subject in need thereof, wherein the pharmaceutical composition further comprises a pharmaceutically acceptable carrier.

16. Use of a TACI-Fc fusion protein in the manufacture of a medicament for treating IgA nephropathy.
